# EUROPÄISCHE PATENTSCHRIFT

(11) **EP 0 589 982 B1**
(45) Veröffentlichungstag und Bekanntmachung des Hinweises auf die Patenterteilung: **17.01.2001**
(21) Anmeldenummer: 92912366.9
(22) Anmeldetag: 13.06.1992
(51) Int. Cl.: C12P 21/08, G01N 33/577, A61K 39/395

(54) **MONOKLONALE ANTIKÖRPER GEGEN DAS HUMANE TNF-BINDENDE PROTEIN I (TNF-BP I)**
MONOCLONAL ANTIBODIES AGAINST HUMAN TNF-BINDING PROTEIN I (TNF-BP I)
ANTICORPS MONOCLONAUX CONTRE LA PROTEINE I DE LIAISON DU FACTEUR DE NECROSE TUMORALE HUMAIN (P I DE LIAISON DU TNF)

(30) Priorität: 19.06.1991 DE 4120213; 03.01.1992 DE 4200049
(43) Veröffentlichungstag der Anmeldung: 06.04.1994
(73) Patentinhaber: Boehringer Ingelheim International GmbH, 55218 Ingelheim am Rhein (DE)
(72) Erfinder: ADOLF, Günther, A-1070 Wien (AT)
(86) Internationale Anmeldenummer: EP9201335
(87) Internationale Veröffentlichungsnummer: WO9222666

(56) Entgegenhaltungen:
- EP-A- 0 334 165
- EP-A- 0 393 438
- EP-A- 0 412 486
- EP-A- 0 444 560
- JOURNAL OF IMMUNOLOGICAL METHODS Bd. 143, 1991, AMSTERDAM Seiten 127 - 136; ADOLF G.R. ET AL: 'A monoclonal antibody-based immunoassay for quantitation of human tumor necrosis factor binding protein I, a soluble fragment of the 60 kDa TNF receptor,in biological fluids'
- PROCEEDINGS OF THE NATIONAL ACADEMY OF SCIENCES OF USA. Bd. 87, April 1990, WASHINGTON US Seiten 3127 - 3131; BROCKHAUS M. ET AL: 'Identification of two types of tumor necrosis factor receptors on human cell lines by monoclonal antibodies' in der Anmeldung erwähnt
- JOURNAL OF EXPERIMENTAL MEDICINE Bd. 172, Oktober 1990, NEW YORK Seiten 1019 - 1023; BETTINA THOMA ET AL: 'Identification of a 60-kD tumor necrosis factor receptor as the major signal transducing component in TNF responses' in der Anmeldung erwähnt

## Beschreibung

Die vorliegende Erfindung bezieht sich auf monoklonale Antikörper gegen das TNF-bindende Protein I (TNF-BP I) und auf Hybridom-Zellinien, die diese sezernieren.

Die zwei strukturell verwandten Zytokine Tumornekrosefaktor (TNF-α) und Lymphotoxin (TNF-β) wurden ursprünglich aufgrund ihrer zytotoxischen in vitro Aktivität gegen Tumorzellen und ihrer Fähigkeit, hämorrhagische Nekrosen von Tumoren in einem Mausmodell zu induzieren, entdeckt. Die Klonierung ihrer cDNAs und deren Expression in *E.coli* haben die Verfügbarkeit dieser Proteine in praktisch unbegrenztem Ausmaß und die Entwicklung hochspezifischer Antikörper und empfindlicher Immunoassays ermöglicht. Es existiert eine Fülle von Information über die biologischen Aktivitäten dieser Proteine, ihre physiologischen Rollen als pleotrope Mediatoren von entzündlichen Prozessen und ihre Beteiligung bei pathologischen Zuständen. Insbesondere wurde eine erhöhte Produktion von TNF-α mit der Pathogenese von z.B. septischem Schock, Gewebeschädigungen bei der "Graft-versus-host"-Erkrankung und der zerebralen Malaria sowie von Kachexie in Zusammenhang gebracht (Beutler, 1988; Paul und Ruddle, 1988; Beutler und Cerami, 1989).

Die möglichen unerwünschten Wirkungen von TNF-α haben zur Suche nach natürlichen Inhibitoren dieses Zytokins veranlaßt. Ein Protein, das an TNF-α bindet und dadurch dessen Aktivität inhibiert, wurde ursprünglich im Harn von Patienten mit Nierenversagen (Peetre et al., 1988) und von Fieberpatienten (Seckinger et al., 1988) identifiziert. Dieses Protein mit einem scheinbaren Molekulargewicht von ca. 30 kDa würde zur Homogenität gereinigt, teilweise sequenziert (EP-A2 308 378) und die cDNA kloniert (Olsson et al., 1989; Engelmann et al., 1989; Himmler et al., 1990; Schall et al., 1990). Die Struktur der cDNA zeigte, daß dieses Protein mit der Bezeichnung TNF-BP das extrazelluläre Fragment eines TNF-Rezeptors (TNF-R) darstellt. (Es wurde angenommen, daß dieses Fragment durch proteolytische Spaltung freigesetzt wird.) Diese Ergebnisse wurden durch die Isolierung des intakten Membranrezeptors für TNF-α, die unabhängig davon von anderen Arbeitsgruppen durchgeführt wurde (Loetscher et al., 1990), bestätigt. Das gesamte Rezeptorprotein besteht aus 455 Aminosäuren (55 - 60 kDa); TNF-BP umfaßt den größten Teil der extrazellulären Domäne des Rezeptors und enthält alle drei N-Glykosylierungsstellen. Es wurde gefunden, daß aus Harn isoliertes TNF-BP am N-Terminus aufgrund proteolytischer Spaltung heterogen ist und daher eine Mischung von zwei molekularen Formen, bestehend aus 161 Aminosäuren (Hauptfraktion) bzw. 172 Aminosäuren, darstellt (Himmler et al., 1990). Kürzlich wurde die Existenz eines zweiten TNF-bindenden Proteins nachgewiesen, das ein Fragment eines zweiten TNF-Rezeptor-Typs mit einem höheren Molekulargewicht (75 - 80 kDa; Engelmann et al., 1990a; Smith et al., 1990; Kohno et al., 1990) darstellt. Die beiden Rezeptoren und Bindungsproteine wurden daraufhin TNF-R I/TNF-BP I und TNF-R II/TNF-BP II (für den 60 kDa bzw. 80 kDa Rezeptor) bezeichnet. Der Sequenzvergleich zeigte, daß die beiden Proteine strukturverwandt sind; insbesondere ist die Anzahl und Verteilung der Cysteinreste sehr ähnlich. Die beiden Rezeptoren unterscheiden sich jedoch immunologisch voneinander (Engelmann et al., 1990a; Brockhaus et al., 1990).

Der humane 60 kDa TNF-Rezeptor spielt eine wesentliche Rolle bei der TNF-α-Signalübertragung. Die Aktivität des Rezeptors ist auf Proteinbasis mehreren regulatorischen Effekten unterworfen: Die Behandlung von Zellen mit Phorbolestern oder anderen Aktivatoren der Proteinkinase C hat eine rasche Verringerung in der Anzahl der zellulären Bindungsstellen für TNF-α zur Folge. Damit geht die Freisetzung des extrazellulären Teils des Rezeptors, entsprechend dem TNF-BP I, durch proteolytische Spaltung einher. Ähnliche Effekte, wenn auch mit unterschiedlicher Kinetik, werden durch verschiedene andere Substanzen, insbesondere durch die physiologischen Liganden TNF-α und TNF-β, hervorgerufen. Die Spaltstellen für die Protease auf dem humanen und dem Ratten-TNF-R I sind konserviert, sie unterscheiden sich hinsichtlich ihrer Struktur von der Spezifität aller bekannten Proteasen. Es ist daher wahrscheinlich, daß ein hochspezifisches proteolytisches Enzym Bestandteil eines Regelkreises ist, der die Empfindlichkeit von Zellen gegenüber TNFs kontrolliert; der genaue Mechanismus dieser Ereignisse ist noch unbekannt. Kürzlich wurde dieses Phänomen auch *in vivo* beobachtet: Es wurde gefunden, daß die Verabreichung von TNF-α an Krebspatienten zu einer deutlichen Zunahme von TNF-BP I im Serum führt (Lantz et al., 1990a).

Die Konzentration von TNF-BP I in Kulturüberständen oder Körperflüssigkeiten ist daher ein Indikator für die Aktivierung des TNF-Rezeptor-Systems *in vitro* bzw. *in vivo* aufgrund von Wechselwirkungen mit den Liganden oder Transmodulation durch andere Mediatoren; die TNF-BP I-Konzentration in Körperflüssigkeiten kann somit als nützlicher Marker für verschiedene Krankheiten angesehen werden.

Es bestand daher der Bedarf nach effizienten, empfindlichen Nachweismethoden für TNF-BP I sowie nach Kits, die für derartige Nachweismethoden einsetzbar sind.

Es sind monoklonale Antikörper gegen TNF-R I beschrieben, die durch Immunisierung mit dem solubilisierten Rezeptor (Brockhaus et al., 1990; EP A2 334 165; Thoma et al., 1990) oder mit gereinigtem TNF-BP I (Engelmann et al., 1990b) hergestellt wurden; von diesen Antikörpern wurde jedoch nicht gezeigt, daß sie sich zur Verwendung in Immuno-Assays für TNF-BP I eignen.

Lantz et al. (1990a) haben einen kompetitiven ELISA entwickelt, bei dem in einer dreistufigen Testmethode mit TNF-BP I beschichtete Testplatten, polyklonale Kaninchenantikörper gegen TNF-BP I, Biotin-markierte Ziegenantikörper gegen Kaninchen-Immunglobulin und Avidin-gekoppelte alkalische Phosphatase verwendet wurden. Mit Hilfe dieses Assays wurden die Gegenwart von TNF-BP I im Serum von normalen Spendern und erhöhte TNF-BP I-Konzentrationen in Seren von Patienten mit Nierenversagen oder von Krebspatienten, die mit TNF-α behandelt wurden, nachgewiesen.

In der EP Al 412 486 werden monoklonale Antikörper gegen TNF-BP I beschrieben. Einer dieser Antikörper wurde in einem Sandwich-ELISA als BeschichtungsAntikörper verwendet; als zweiter Antikörper wurden polyklonale Kaninchen-Anti-TNF-BP-I-Antikörper, als dritter, enzymgekoppelter Antikörper polyklonale Ziegen-anti-Kaninchen-Antikörper verwendet.

Die vorbekannten Assays sind vom Aufbau und der Durchführung her kompliziert, außerdem bedingt der Einsatz polyklonaler Antikörper die Verwendung von Tieren, die zu vermeiden man zunehmend bestrebt ist.

Der vorliegenden Erfindung lag die Aufgabe zugrunde, monoklonale Antikörper mit Spezifität für humanes TNF-BP I bereitzustellen, die sich zur Verwendung in einem einfach durchzuführenden, hochempfindlichen Immunoassay zum Nachweis von TNF-BP I eignen.

Die vorliegende Erfindung betrifft monoklonale Antikörper gegen humanes TNF-BP I der Bezeichnung tbp-1, tbp-2 und tbp-6, aktive Fragmente davon sowie die Hybridom-Zellinien TBP-1, TBP-2 und TBP-6, die diese Antikörper produzieren.

Die Hybridomzellinien mit der Bezeichnung TBP-1 und TBP-2 wurden am 5. Juni 1991, die Zellinie mit der Bezeichnung TBP-6 am 28. November 1991 bei der European Collection of Animal Cell Cultures (ECACC; Salisbury, Vereinigtes Königreich) nach dem Budapester Vertrag über die Hinterlegung von Mikroorganismen für Patentzwecke hinterlegt (TBP-1: Hinterlegungsnummer 91060555, TBP-2: Hinterlegungsnummer 91060556, TPB-6: Hinterlegungsnummer 91112811).

Die erfindungsgemäßen Hybridom-Zellinien wurden erhalten, indem Mäuse mit aus menschlichem Harn hochgereinigtem TNF-BP I (Olsson et al., 1989) nach an sich bekannten Methoden immunisiert und Milzzellen von Mäusen mit positiver Antikörper-Reaktion zur Fusionierung mit Myelomzellen verwendet wurden, um Hybridomzellen zu erhalten, die monoklonale Antikörper gegen TNF-BP I sezernieren. Die erste Zellfusion ergab zwei Kulturen, die monoklonale Antikörper gegen TNF-BP I produzieren; eine zweite Zellfusion ergab eine dritte Antikörper produzierende Kultur. Die erhaltenen Antikörper wurden mit tbp-1, tbp-2 und tbp-6 bezeichnet. Die Antikörper wurden gereinigt und charakterisiert:
tbp-1 und tbp-6 sind IgGl-Antikörper, tbp-2 ist ein IgG2b-Antikörper. Alle drei Antikörper waren in der Lage, TNF-BP I in Western Blots zu erkennen, wobei tbp-1 die stärkste Reaktivität zeigte. tbp-2 reagierte schwächer, tbp-6 ergab die schwächste Färbung. Zur Charakterisierung der Epitope, die von den Antikörpern erkannt werden, wurden die drei Antikörper sowie ein vierter Antikörper mit der Bezeichnung H398, der durch Immunisierung mit solubilisiertem TNF-Rezeptor erhalten worden war (Thoma et al., 1990), untersucht. Die untersuchten Antikörper erkennen drei verschiedene Epitope auf dem TNF-BP I-Molekül, wobei tbp-2 und H398 dasselbe oder überlappende Epitope erkennen. Die in Gegenwart von TNF-α in verschiedenen Anordnungen mit den Antikörpern durchgeführten Sandwich-ELISAs lassen den Schluß zu, daß die Epitope, die von H398 und tbp-2 erkannt werden, an der Bindung von TNF-α beteiligt sind, während die von tbp-1 und tbp-6 erkannten mit der Ligandenbindungsstelle nicht in Beziehung stehen. Es wurde überraschend festgestellt, daß bestimmte monoklonale Antikörper gegen TNF-BP I nicht nur TNF-BP I in Gegenwart von überschüssigem TNF-α binden können, sondern daß sie darüberhinaus die Schutzwirkung von TNF-BP I gegen die zytotoxische Aktivität von TNF-α und TNF-β deutlich erhöhen. Dieser Effekt wurde bei den beiden Antikörpern tbp-1 und tbp-6 beobachtet. (Diese beiden Antikörper gehören zur Gruppe von Antikörpern gegen TNF-BP I, die nicht mit TNF-α und TNF-β um die Bindung von TNF-BP I konkurrieren. Im Gegensatz dazu blockieren die Antikörper tbp-2 und H398, die mit TNF-α um die Bindung an TNF-BP I konkurrieren, die Wirkung von TNF-BP I.)

Die vorliegende Erfindung betrifft in einem weiteren Aspekt die Verwendung von tbp-1 und/oder tbp-2 zum Nachweis von TNF-BP I in Körperflüssigkeiten oder Zellkulturüberständen mittels Immunoassay. (Unter die Bezeichnung tbp-1 oder tbp-2 fallen im Zusammenhang mit der Verwendung dieser Antikörper im folgenden auch ihre aktiven Fragmente, die an TNF-BP I binden. Dem Fachmann auf dem einschlägigen Gebiet sind Methoden zur Herstellung aktiver Antikörper-Fragmente (Fab-Fragmente), z.B. mittels Enzymverdau, geläufig.)

Gegenstand der Erfindung ist außerdem die Verwendung von tbp-1 und tbp-6 zum Nachweis von TNF-BP I in Körperflüssigkeiten oder Zellkulturüberständen mittels Immunoassay. Die Antikörperkombination tbp-1/tbp-6 ist besonders geeignet für die Anwendung bei Proben, in denen sehr hohe TNF-α- und/oder TNF-β-Konzentrationen vorliegen, die die Bestimmung von TNF-BP I in anderen Testsystemen stören.

Die im Rahmen der vorliegenden Erfindung anwendbaren Immunoassays beruhen auf Standardmethoden, die dem Fachmann auf dem einschlägigen Gebiet geläufig sind und von denen ein großes Angebot zur Verfügung steht. Diese Methoden basieren auf der Bildung eines Komplexes zwischen der zu bestimmenden antigenen Substanz und einem oder mehreren Antikörpern. Einer oder mehrere der Komplexpartner ist dabei markiert, sodaß das Antigen nachgewiesen und/oder quantitativ bestimmt werden kann. Die Markierung kann z.B. in Form eines gekoppelten Enzyms, Radioisotops, Metallchelats, oder einer fluoreszierenden, chemilumineszierenden oder biolumineszierenden Substanz vorliegen.

Im Falle eines kompetitiven Immunoassays konkurriert das Antigen in der zu analysierenden Probe mit einer bekannten Menge von markiertem Antigen um die Bindung an die Antikörper-Bindungstellen. Die Menge von markiertem Antigen, gebunden an den Antikörper, ist daher verkehrt proportional zur Antigenmenge in der Probe.

Bei Tests, in denen markierte Antikörper verwendet werden, ist die Menge an markiertem, gebundenem Antikörper direkt proportional zur Menge an Antigen.

Assays, die auf der Bildung eines Antikörper/Antigen/Antikörper-Komplexes basieren, wobei zwei Antikörper eingesetzt werden, die einander bei der Bindung an das Antigen nicht behindern, werden als "Sandwich"-Immunoassays bezeichnet.

Da monoklonale Antikörper in unbegrenzter Menge in konstanter Qualität verfügbar sind, weisen Immunoassays unter Verwendung monoklonaler Antikörper aufgrund ihrer konstanten Qualität und Reproduzierbarkeit gegenüber Assays, die sich polyklonaler Antikörper bedienen, entscheidende Vorteile auf. Außerdem wird der mit polyklonalen Antikörpern verbundene Nachteil, für deren Herstellung laufend Tiere einsetzen zu müssen, vermieden.

Bevorzugt werden die erfindungsgemäßen monoklonalen Antikörper in einem Sandwich-Immunoassay, insbesondere in einem Sandwich-ELISA verwendet.

Die erfindungsgemäßen monoklonalen Antikörper sind in Sandwich-Immunoassays als Beschichtungs- und Markierungs-gekoppelte Antikörper einsetzbar und machen somit den Einsatz polyklonaler Antikörper überflüssig.

In einem weiteren Aspekt betrifft die vorliegende Erfindung Immunoassay-Kits, insbesondere Sandwich-Immunoassay-Kits, die tbp-1 und/oder tbp-2 enthalten.

Eine bevorzugte Ausführungsform der vorliegenden Erfindung ist ein Sandwich-Immunoassay-Kit, vorzugsweise ein Sandwich-ELISA-Kit, in dem tbp-1 den Beschichtungsantikörper und tbp-2 den markierten, vorzugsweise enzymgekoppelten Antikörper darstellt.

Es ist im Rahmen der vorliegenden Erfindung möglich, in einem Sandwich-Immunoassay tbp-1 oder tbp-2 durch einen anderen Antikörper zu ersetzen, der in der Lage ist, mit tbp-2 bzw. tbp-1 einen Antikörper/Antigen/Antikörper-Komplex zu bilden.

Im Falle des Ersatzes von tbp-1 oder tbp-2 durch einen anderen Antikörper wird bevorzugt ein Antikörper verwendet, der jeweils dasselbe Epitop von TNF-BP I oder einen Bereich davon erkennt wie der zu ersetzende tbp-1 bzw. tbp-2.

Antikörper, die sich in dem Immunoassay aufgrund ihrer Fähigkeit, mit tbp-2 bzw. tbp-1 einen Antikörper/Antigen/Antikörper-Komplex zu bilden, als Ersatz für tbp-1 oder tbp-2 eignen, können mit Hilfe von Sandwich-Immunoassays ermittelt werden. Dabei werden Immunoassay-Platten mit tbp-1 oder tbp-2 beschichtet, das Antigen aufgegeben und die zu untersuchenden, markierten Antikörper aufgetragen. Antikörper, die mit tbp-1 bzw. tbp-2 einen Antikörper/Antigen/Antikörper-Komplex bilden können, was durch Messung der Markierung des Testantikörpers feststellbar ist, erkennen ein anderes Epitop auf dem Antigen als tbp-1 bzw. tbp-2. Antikörper, die nicht in der Lage sind, ein Sandwich mit tbp-1 bzw. tbp-2 zu bilden, weisen dieselbe oder überlappende Epitoperkennung auf wie diese Antikörper.

Im Falle des Ersatzes eines der Antikörper tbp-1 oder tbp-2 im Sandwich-Immunoassay wird bevorzugt der Markierungs-gekoppelte Antikörper tbp-2 durch einen Antikörper ersetzt, der dieselbe oder überlappende Epitop-Spezifität aufweist wie tbp-2. Ein Beispiel für einen geeigneten Antikörper ist der von Thoma et al. (1990) beschriebene monoklonale Antikörper H398, von dem im Rahmen der vorliegenden Erfindung gezeigt werden konnte, daß er an ein identisches oder überlappendes Epitop bindet.

Mit Hilfe eines erfindungsgemäßen Sandwich-ELISAs auf Basis tbp-1/tbp-2 konnte TNF-BP I in Humanserum, Plasma, Harn und Zellkulturüberständen bei einer Empfindlichkeit von ca. 200 ng/l und einer Genauigkeit von mehr als 10 % nachgewiesen werden.

Es wurde überraschend festgestellt, daß die natürlichen Liganden für den TNF-Rezeptor, TNF-α und TNF-β, die Aussagekraft des erfindungsgemäßen Immunoassays, in dem tbp-1 und tbp-2 eingesetzt werden, nicht beeinträchtigen: TNF-β übt keinen meßbaren Einfluß auf den Assay aus, TNF-α zeigt erst bei Konzentrationen von ≥10 µg/l eine meßbare Änderung des Signals. Da die TNF-α-Konzentrationen in gesunden Personen für gewöhnlich ≤20 ng/l betragen und selbst bei ernsten pathologischen Zuständen die TNF-α-Konzentrationen nur in seltenen Fällen 1 µg/l übersteigen (z.B. Lähdevirta et al., 1988; Offner et al., 1990), kann eine Beeinträchtigung des erfindungsgemäßen Immunoassays durch unter natürlichen Bedingungen ausgeschütteten endogenen TNF-α ausgeschlossen werden.

Aufgrund seiner Empfindlichkeit ist ein Immunoassay auf Basis der monoklonalen Antikörper tbp-1/tbp-2 auch geeignet, nach unten von den Normwerten abweichende Konzentrationen von TNF-BP I nachzuweisen. Damit können Funktionsstörungen des Organismus diagnostisch erfaßt werden, die mit einer verminderten Produktion von TNF-BP I einhergehen.

Außer für den Nachweis von TNF-BP I in Serum, Plasma und Harn sowie in Zellkulturüberständen können die monoklonalen Antikörper tbp-1 und tbp-2 auch für den Nachweis von TNF-BP I in anderen Körperflüssigkeiten, z.B. in der Zerebrospinalflüssigkeit oder in Bronchoalveolarsekreten, verwendet werden.

In den im Rahmen der vorliegenden Erfindung durchgeführten Versuchen wurde überraschend festgestellt, daß das in einem Sandwich-ELISA mit der Kombination tbp-1/tbp-6 erhaltene Signal nicht einmal durch extrem hohe TNF-α- bzw. TNF-β-Konzentrationen im Bereich von 10 mg/l beeinträchtigt wurde.

Die Antikörperkombination tbp-1/tbp-6 wird bevorzugt in einem Sandwich-Immunoassay, insbesondere in einem Sandwich-ELISA, eingesetzt, wobei tbp-1 und tbp-6 gleichermaßen als Beschichtungsantikörper und markierter Antikörper verwendet werden können.

Beispiele für die Anwendung des Antikörperpaares tbp-1/tbp-6 zum Nachweis von TNF-BP I sind Proben aus in vitro Experimenten, in denen Zellen, z.B. Leukozyten, mit Lipopolysacchariden stimuliert wurden, oder Serumproben von Versuchstieren, die mit großen Mengen von Lipopolysacchariden oder Bakterien behandelt wurden. Unter diesen Bedingungen wird TNF-α in extrem hohen Mengen ausgeschüttet.

Mit Hilfe der vorliegenden Erfindung wird somit eine hochempfindliche Nachweismethode für TNF-BP I bereitgestellt, mit der die Aktivierung des TNF-Rezeptors durch seine physiologischen Liganden TNF-α und TNF-β sowie seine Transmodulation durch andere Mediatoren in verschiedenen pathologischen Zuständen oder in *in vitro* Modellen festgestellt werden kann. Der Nachweis von TNF-BP I in Körperflüssigkeiten dient somit vor allem zur Diagnose von pathologischen Zuständen, die mit einer erhöhten TNF-α-Produktion einhergehen bzw. zur Absicherung solcher Diagnosen.

Ein Vorteil der TNF-BP I-Bestimmung gegenüber der TNF-α-Bestimmung ergibt sich dadurch, daß von TNF-BP I Normalwerte im Serum gefunden werden, wodurch eine Bestimmung auch geringfügig erhöhter Werte und damit eine Diagnose möglich ist.

Trotz des deutlichen Zusammenhanges zwischen der Induktion von TNF-α und dem Auftreten von septikämischen und endotoxämischen Reaktionen in Tieren brachte die Bestimmung von TNF-α in schwer an gram-negativen Infektionen erkrankten Patienten widersprüchliche Ergebnisse. Dies dürfte mit den Mechanismen in Zusammenhang stehen, die die Synthese und Ausscheidung von TNF-α kontrollieren. Nach Anregung durch einen geeigneten Stimulus wird TNF-α rasch von Makrophagen ausgeschüttet, im Anschluß daran dürften die Makrophagen gegen weitere Stimulierung resistent sein. Außerdem ist die Halbwertszeit im Plasma kurz, sie beträgt im Menschen nur 15 bis 17 min. Diese Phänomene dürften die Ursache dafür sein, daß das Auftreten von TNF-α im Blutkreislauf rasch und kurzlebig und daher schwierig nachweisbar ist (Michie et al., 1988). Wenn dem Patienten daher nicht rechtzeitig Blut abgenommen wird, ist es möglich, daß zum Zeitpunkt der Analyse die Erhöhung der TNF-α-Konzentration nicht mehr nachweisbar ist. Von Lantz et al. (1990a) wurde festgestellt, daß nach Infusion mit TNF-α der TNF-BP I-Spiegel im Serum wesentlich langsamer abfällt als der Serum-TNF-Spiegel. Dieser Befund zeigt, daß die Bestimmung von TNF-BP I-Konzentrationen als Grundlage für eine Diagnose insbesondere dann von Vorteil ist, wenn pathologische Zustände diagnostiziert werden sollen, die mit einer Aktivierung des TNF-Rezeptor-Systems, insbesondere durch TNF-α, verbunden sind und bei denen der TNF-α-Spiegel im Vergleich zum TNF-BP I-Spiegel rascher absinkt.

Beispiele für Erkrankungen, für deren Diagnose die Bestimmung von TNF-BP I herangezogen werden kann, sind gram-negative oder allgemeine bakterielle Infektionen, septischer Schock, Gewebeschädigungen bei der "Graft-versus-host"-Erkrankung und der zerebralen Malaria sowie Kachexie.

Die erfindungsgemäßen Immunoassays sind insbesondere bei der Diagnose von septischem Schock, der bei nicht rechtzeitig vorgenommener Therapie eine lebensbedrohende Erkrankung darstellt, von Vorteil.

Mit Hilfe eines erfindungsgemäßen Immunoassays auf Basis der monoklonalen Antikörper tbp-1 und tbp-2 konnte TNF-BP I im Serum von normalen Spendern bei einer Durchschnittskonzentration von ca. 2 µg/l nachgewiesen werden. Erhöhte Werte wurden im Serum von Patienten mit schweren Verbrennungen bestimmt; sehr hohe Werte wurden in Dialysepatienten nachgewiesen, während die Seren von Patienten mit chronischer Polyarthritis keine erhöhten TNF-BP I-Konzentrationen aufwiesen.

Mit den erfindungsgemäßen Immunoassays wird außerdem erstmals eine einfache immunologische Nachweismethode für TNF-BP I im Harn bereitgestellt; in Harnproben von normalen Spendern wurde eine TNF-BP I-Durchschnittskonzentration von ca. 2 µg/l bestimmt.

Die erfindungsgemäßen Immunoassays können somit zur Diagnose von pathologischen Zuständen, bei denen eine Korrelation von erhöhten TNF-BP I-Konzentrationen im Harn und im Serum besteht, wie sie bei Nierenversagen festgestellt wurde, eingesetzt werden, indem die TNF-BP I-Konzentration im Harn bestimmt wird. Diese Methode bietet den Vorteil, auf eine Blutabnahme verzichten und die Diagnose aufgrund der für den Patienten wesentlich angenehmeren Harnanalyse erstellen zu können.

Unter den Begriff "Diagnose", für die die erfindungsgemäßen Immunoassays verwendet werden können, fällt auch die Überwachung des Verlaufs einer Erkrankung, die mit einer Aktivierung des TNF-Rezeptorsystems verbunden ist, bzw. des Verlaufs der Therapie zur Behandlung einer solchen Erkrankung, z.B. einer Therapie mit Antikörpern gegen TNF-α. Die Verwendung des erfindungsgemäßen Immunoassays ist weiters zweckmäßig für die Überwachung des Verlaufs von Therapien, bei denen TNF-α oder TNF-β verabreicht werden. Im Zuge dieser diagnostischen Anwendungen wird im allgemeinen in regelmäßigen Zeitabständen die Probe einer Körperflüssigkeit aus dem Patienten entnommen und deren Gehalt an TNF-BP I bestimmt.

Monoklonale Antikörper gegen TNF-BP I, die nicht mit TNF-α und/oder TNF-β um die Bindung an TNF-BP I konkurrieren und die die Schutzwirkung von TNF-BP I erhöhen, können erfindungsgemäß verwendet werden, um die Wirkung von TNF-BP I im Rahmen der Behandlung von Erkrankungen, bei denen eine schädigende Wirkung von TNF-α und/oder TNF-β auftritt, zu verstärken. (Es wurde festgestellt, daß die Schutzwirkung von TNF-BP I gegenüber TNF-α relativ schwach ist (Lantz et al., 1990b, Loetscher et al., 1991).)

Um die Verstärkung der Schutzwirkung dieser Antikörper sowohl für endogenes TNF-BP I als auch für therapeutisch verabreichtes exogenes TNF-BP I auszunutzen, können die Antikörper für sich oder in Kombination mit TNF-BP I in geeigneten Zubereitungen als Therapeutikum eingesetzt werden.

Beispiele für solche Antikörper, die die Schutzwirkung von TNF-BP I gegen TNF-α und TNF-β verstärken, sind tbp-1 und tbp-6. Die diesbezügliche Eignung anderer Antikörper gegen TNF-BP I kann festgestellt werden, indem die Antikörper in Bioassays auf ihre Wirkung auf TNF-BP I untersucht werden. Die Dosierung der monoklonalen Antikörper wird nach der TNF-BP I-Dosis bemessen, sie liegt, bezogen auf die TNF-BP I-Menge, zweckmäßig im Bereich von äquimolar bis ca. 100facher molarer Überschuß.

Ein weiteres Anwendungsgebiet der vorliegenden Erfindung ist die Verwendung der monoklonalen Antikörper tbp-1, tbp-2 und tbp-6 in immobilisierter Form für die Reinigung von TNF-BP I mittels Affinitätschromatographie.

Weiters können die erfindungsgemäßen monoklonalen Antikörper, insbesondere tbp-1, für den Nachweis von TNF-BP I in Immunoblots verwendet werden.

### Figurenübersicht

- Fig. 1:: ELISAs für TNF-BP I unter Verwendung monoklonaler Antikörper
- Fig. 2:: Repräsentative Eichkurven für TNF-BP I ELISAs von Serum- und Harnproben
- Fig. 3:: Einfluß von TNF-α auf die Immunreaktivität von TNF-BP I
- Fig. 4:: TNF-BP I-Konzentrationen in Humanserum und Harn
- Fig. 5:: Wirkung von TNF-α auf den Nachweis von TNF-BP I in Sandwich-ELISAs
- Fig. 6:: Einfluß der monoklonalen Antikörper auf die Schutzwirkung von TNF-BP I im zytotoxischen Bioassay

Die Erfindung wird durch die folgenden Beispiele näher erläutert:

### Beispiel 1

Herstellung von monoklonalen Antikörpern mit Spezifität für humanes TNF-BP I

### a) Immunisierung

3 ca. 6 Wochen alte weibliche BALB/c Mäuse wurden mit dem nach der in der EP A2 393 438 beschriebenen Methode zur Homogenität gereinigten TNF-BP I nach folgendem Schema immunisiert:

| | | |
|---|---|---|
| 1. | Immunisierung | 9 µg TNF-BP I pro Maus in komplettem Freund'schem Adjuvans, intraperitoneal |
| 2. | Immunisierung | 9 µg TNF-BP I pro Maus in inkomplettem Freund'schem Adjuvans, intraperitoneal, 3 Wochen nach der 1. Immunisierung |
| 3. | Immunisierung | 9 µg TNF-BP I pro Maus in inkomplettem Freund'schem Adjuvans, intraperitoneal, 5 Wochen nach der 2. Immunisierung. |

8 Tage danach wurden den Mäusen Serumproben entnommen und mittels eines Sandwich-ELISA auf die Bildung von Antikörpern gegen TNF-BP I untersucht. Dazu wurde TNF-BP I an mit Kaninchenantikörpern gegen TNF-BP I beschichtete Testplatten gebunden und spezifische Antikörper mit Peroxidase-gekoppelten Kaninchenantikörpern gegen Mäuse-Immunglobulin nachgewiesen. Die Testseren wurden in Verdünnungen von 1:10², 1:10³, 1:10⁴ und 1:10⁵ aufgelegt. Es zeigten alle drei Mäuse positive Reaktion (Absorption mehr als zweifacher Untergrund) bei bis zu 10⁵facher Verdünnung. Die Maus mit dem höchsten Titer wurde ca. 8 Wochen nach der 3. Immunisierung an drei aufeinanderfolgenden Tagen mit je 4 µg TNF-BP I in PBS geboostert; die Milzzellen dieser Maus wurden am folgenden Tag für die Fusion mit Hybridomzellen verwendet.

In ähnlicher Weise wurde eine zweite Immunisierung durchgeführt mit dem Unterschied, daß die dazu verwendete Maus zusätzlich 8 Monate nach der dritten Immunisierung eine vierte Dosis TNF-BP I (15 µg) erhielt und 7 Wochen danach geboostert wurde.

### b) Fusion:

Die Milz der Maus wurde einen Tag nach der letzten Injektion steril entnommen, mechanisch zerkleinert und mit serumfreiem Kulturmedium (RPMI 1640) gewaschen. Ca. 10⁸ Milzzellen wurden nach der Methode von Köhler und Milstein (1975) in Gegenwart von PEG 4000 (Merck, 40% in serumfreiem Kulturmedium) mit ca. 5x10⁷ P3X63Ag8.653 BALB/c-Myelomzellen (Kearney et al., 1979) fusioniert. Danach wurden die Zellen in HAT-Selektionsmedium (RPMI 1640 - komplettiert mit 100 U/ml Natrium-Penicillin G, 50 U/ml Streptomycin und 20 % FCS - mit 10⁻⁴ M Hypoxanthin, 4x10⁻⁷ M Aminopterin und 1.6x10⁻⁵ M Thymidin) suspendiert und in 16 96-Loch-Mikrotiter-Platten, die peritoneale Mäusezellen als "Feeder Layer" enthielten, verteilt. Nach 11 Tagen wurden Überstände entnommen und auf Antikörperproduktion getestet. Von den 1500 Kulturen, die in selektives Medium ausgesät wurden, zeigten mehr als 90 % Wachstum von Hybridomen.

### c) Screening von Hybridom-Kulturüberständen

Sofern nicht anders angegeben, wurden für alle ELISA-Versuche die folgenden Puffer verwendet:
Beschichtungspuffer: 0.05 M Natriumcarbonat pH 9.6
Waschmedium: Phosphat-gepufferte Kochsalzlösung pH 7.4 (PBS), enthaltend Tween 20 zu 0.5 g/l
Testpuffer: PBS mit Rinderserumalbumin (5 g/l) und Tween 20 (0.5 g/l)
Substratlösung: Tetramethylbenzidindihydrochlorid (0.1 g/l) und Natriumperborat (0.05 g/l) in 0.05 M Kaliumcitrat pH 5.0
Stopp-Lösung: 2 M Schwefelsäure

96-Loch-Immunoassay-Platten wurden mit Kaninchenantikörpern gegen TNF-BP I, teilweise gereinigt durch Ammoniumsulfat-Fällung (50 % Sättigung), in Beschichtungspuffer bei einer Konzentration entsprechend einer 1:3000 Serumverdünnung (50 µl/Vertiefung, Inkubation über Nacht bei 4°C oder während einer Stunde bei 37°C) beschichtet. Die Platten wurden einmal gewaschen und eine Stunde lang mit Testpuffer bei Raumtemperatur blockiert. Daraufhin wurden Hybridomüberstände (50 µl) zusammen mit dem Antigen (50 µl, 10 µg TNF-BP I/1 in Testpuffer) zugegeben und 2 h lang bei Raumtemperatur inkubiert. Die Platten wurden einmal gewaschen, eine Lösung von Peroxidase-gekoppelten Kaninchenantikörpern gegen Maus-Immunglobuline (DAKO, Dänemark, Verdünnung 1:5000 im Testpuffer, 50 µl/Vertiefung) hinzugefügt und 2 h lang bei Raumtemperatur bebrütet. Daraufhin wurden die Platten 3 x gewaschen und in jede Vertiefung 200 µl Substratlösung gegeben. Nach 20 bis 40 min wurde die Reaktion durch Hinzufügen von 50 µl Stopp-Lösung unterbrochen. Die Absorption der Lösung wurde in einem ELISA-Reader bei einer Wellenlänge von 450 nm (Referenz: 690 nm) gemessen, wobei Kulturmedium als negative und verdünntes Mausimmunserum als positive Kontrolle verwendet wurden.
Nur zwei von den untersuchten Kulturen aus der ersten Immunisierung zeigten Antikörperproduktion (TBP-1 und TBP-2); die zweite Fusion ergab eine dritte Antikörper-positive Zellinie (TBP-6).

Die positiven Kulturen wurden nach ca. 25 Tagen von HAT-Medium in HT-Medium überführt, nach weiteren 10 Tagen auf normales Kulturmedium (RPMI 1640 komplett, 1 % Antibiotika, 10 % L-Glutamin, 10 % FCS) umgestellt.

### d) Klonierung der Hybridome:

Die positiven Kulturen wurden nach der Methode der limitierenden Verdünnung ("limiting dilution") kloniert. Dabei wurde derart verdünnt, daß 100 µl Kulturmedium 1 Zelle enthielten, worauf die Vertiefungen einer 96-Loch-Platte mit diesem Volumen beschickt wurden (es wurden insgesamt 3 Platten angesetzt, die am Vortag je Vertiefung mit 100 µl Maus-Peritoneal-Makrophagensuspension beschickt worden waren). Die Kulturüberstände wurden mittels ELISA, wie oben beschrieben getestet; positive Klone jeder Kultur wurden gepoolt, expandiert und eingefroren.

### e) Produktion monoklonaler Antikörper

Zur Produktion von Antikörpern in vivo wurden von den Hybridomkulturen je ca. 10⁷ Zellen intraperitoneal in BALB/c Mäuse injiziert, die 2 bzw. 3 Tage vorher mit 0.5 ml inkomplettem Freund'schem Adjuvans konditioniert worden waren. Nach ca. 10 bis 14 Tagen wurde die Ascitesflüssigkeit entnommen. Die gebildeten monoklonalen Antikörper wurden nach Ammonsulfatfällung mittels Affinitätschromatographie über trägergebundenes Protein-G gereinigt.

Für die Antikörperproduktion in Zellkultur wurde fötales Kälberserum (FCS) durch Chromatographie auf Protein-G-Sepharose gereinigt, um Rinder-IgG zu entfernen; dieses Serumpräparat wurde in einer Konzentration von 5 % für die Hybridomkulturen verwendet. Aus den Kulturüberständen wurden die Antikörper erneut über Protein-G-Sepharose isoliert. Alternativ wurden die Zellen in serumfreiem Medium (Serum-free and protein-free hybridoma medium, Fa. SIGMA, Kat.Nr. 5-2772; USA) gezüchtet und die Antikörper ebenso durch Chromatographie an Protein-G-Sepharose isoliert.

### Beispiel 2

### Charakterisierung der monoklonalen Antikörper

Die Antikörper-Subisotypen wurden unter Verwendung von Peroxidase-gekoppelten Kaninchenantikörpern (Serotec, Oxford, GB) bestimmt: tbp-1 und tbp-6 sind IgGl-Antikörper, tbp-2 ist ein IgG2b-Antikörper.

Im Western Blot erkannten alle drei Antikörper TNF-BP I; tbp-1 zeigte starke Reaktivität, tbp-2 reagierte schwächer, tbp-6 ergab nur eine sehr schwache Färbung.

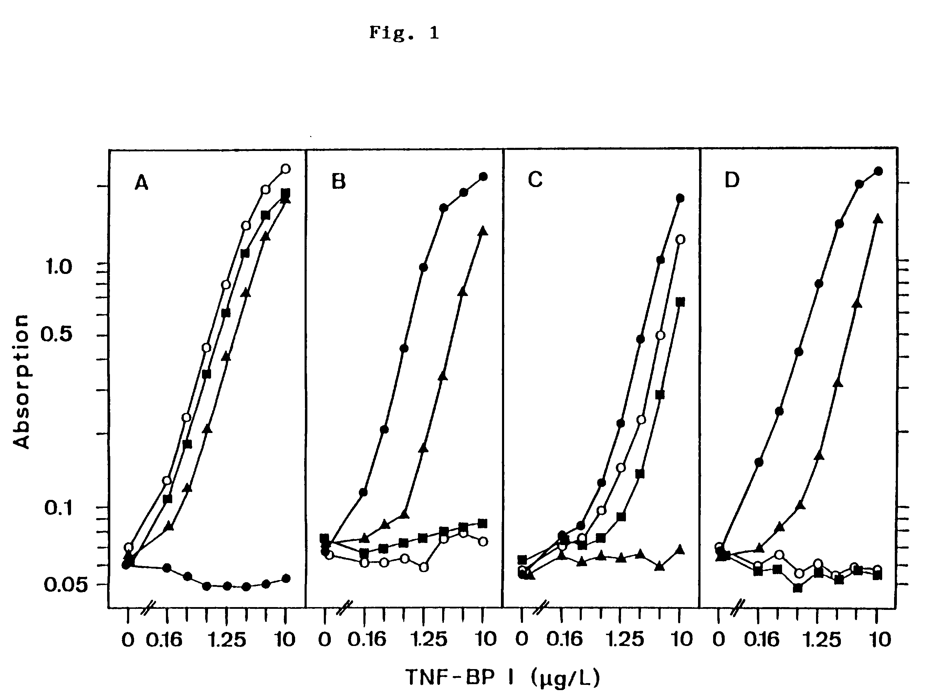

Zur Bestimmung der Epitope, die von den Antikörpern erkannt werden, wurden die drei Antikörper tbp-1, tbp-2 und tbp-6 sowie der von Thoma et al.(1990) durch Immunisierung mit solubilisiertem TNF-Rezeptor entwickelte Antikörper H398 an Meerrettich-Peroxidase gekoppelt und mittels ELISA charakterisiert: Testplatten wurden jeweils mit einem der unmarkierten Antikörper (10 mg/l) beschichtet, worauf eine Verdünnungsserie des Antigens zugegeben und anschließend jeweils einer der Enzym-gekoppelten Antikörper aufgetragen wurde. Dieser Versuch wurde in allen möglichen Anordnungen durchgeführt [Fig. 1: A: tbp-1; B: tbp-2, C: tbp-6, D: H398. Die Symbole zeigen die Peroxidase-Konjugate: tbp-1 (gefüllte Kreise), tbp-2 (gefüllte Quadrate), tbp-6 (gefüllte Dreiecke), H398 (offene Kreise)]. Es zeigte sich, daß keine der einzelnen Antikörper-Spezies in der Lage war, ein "Sandwich" zu bilden, was darauf hindeutet, daß das Antigen als Monomer vorliegt. Kombinationen der Antikörper tbp-1 mit tbp-2, tbp-6 oder H398 sowie Kombinationen von tbp-2 mit tbp-6 oder tbp-6 mit H398 waren in der Lage, dosisabhängige Signale zu geben, während tbp-2 in Kombination mit H398 nicht reagierte. Daraus wurde gefolgert, daß die Antikörper drei verschiedene Epitope auf dem TNF-BP I-Molekül erkennen; tbp-2 und H398 binden an identische oder überlappende Epitope. Für die weitere Entwicklung wurde eine Testanordnung gewählt, in der tbp-1 den Beschichtungsantikörper und tbp-2 den Peroxidase-gekoppelten Antikörper darstellt.

### Beispiel 3

### Entwicklung eines Enzyme Linked Immunosorbent Assay (Sandwich-ELISA)

Im Hinblick auf die Anwendung der ELISAs zum Nachweis von TNF-BP I in Humanserum wurden zunächst verschiedene Medien auf ihre Eignung als Verdünnungsmedium für Proben und Standard untersucht. Während sowohl FCS als auch Kälberserum brauchbare Dosis-Wirkungskurven ergaben, zeigte gepooltes normales Humanserum einen sehr hohen Leerwert. Um zu überprüfen, ob dieses Phänomen auf unspezifische Reaktivität oder auf die Gegenwart von Antigen zurückzuführen ist, wurde humanes Serum über eine Affinitätssäule, enthaltend immobilisierten TNF-α, geschickt. Als der Durchfluß der Chromatographiesäule als Verdünnungsmedium verwendet wurde, betrug der Leerwert etwa gleich viel wie mit Kälberserum. Dies deutete darauf hin, daß normales Humanserum immunreaktives und biologisch aktives TNF-BP I enthält. Die Konzentration von TNF-BP I in dem verwendeten Serum-Pool wurde auf ca. 1 µg/l geschätzt. Standardkurven, die mit 50 % Kälberserum erstellt wurden, waren von denen mit 50 % Humanserum, aus dem TNF-BP I entfernt worden war, nicht zu unterscheiden. Daher wurde das erstere Medium für alle folgenden Versuche verwendet (von allen verwendeten Lieferungen von Kälberserum, die von verschiedenen Firmen bezogen wurden, erwiesen sich nur zwei als geeignet; alle anderen zeigten niedrige Wiederfindung).

Die Etablierung und Durchführung der Tests erfolgte nach Standardmethoden.

Zunächst wurde in Vorversuchen für die Entwicklung eines Assays zur Bestimmung von TNF-BP I in Serum festgestellt, daß ein sog. "zweistufiger" Assay, das ist eine Methode, in der an die Inkubation der Probe ein Waschschritt angeschlossen wird und die Inkubation mit dem Antikörper-Enzymkonjugat getrennt durchgeführt wird, keinen Vorteil gegenüber der schnelleren und bequemeren "einstufigen" Methode bringt. In Vorversuchen wurde weiters die Konzentration des Beschichtungs-Antikörpers variiert, ebenso die Inkubationszeit für die Immunreaktion.

Der optimierte Assay wurde für die Bestimmung von TNF-BP I im Serum wie folgt durchgeführt: 96-Loch-Immunoassayplatten wurden mit dem monoklonalen Antikörper tbp-1 bei einer Konzentration von 3 mg/l in Beschichtungspuffer beschichtet (über Nacht bei 4°C oder 1 h lang bei Raumtemperatur; 100 µl/Vertiefung). Die Vertiefungen wurden einmal gewaschen und die verbleibenden Bindungsstellen mit 200 µl Testpuffer 1 h lang bei Raumtemperatur blockiert. Nach einem weiteren Waschzyklus wurden die Vertiefungen der Reihen 2 und 11 mit 100 µl 50 % Kälberserum/50 % PBS befüllt. Eine Standardlösung TNF-BP I (vgl. Beispiel 1a, 20 µg/l in 50 % Kälberserum/50 % PBS, 100 µl) wurde in die Vertiefungen A2 und All pipettiert; serielle Verdünnungen im Verhältnis 1:2 wurden in den Reihen 2 und 11 direkt in den Vertiefungen vorgenommen. Alle anderen Vertiefungen erhielten 50 µl PBS, und die Serumproben wurden jeweils zweifach auspipettiert (50 pl/Vertiefung). In alle Vertiefungen wurden 50 µl einer Lösung von Peroxidase-gekoppeltem Antikörper tbp-2 in Testpuffer gegeben, nachdem in Vorversuchen die geeignete Verdünnung bestimmt worden war. (Die Kopplung der Antikörper mit Meerrettich-Peroxidase (Boehringer Mannheim) wurde nach der von Wilson und Nakane (1978) beschriebenen Methode durchgeführt.) Die Platten wurden 3 h lang auf einem Platten-Schüttelgerät bei Raumtemperatur inkubiert. Die Platten wurden daraufhin 3 x gewaschen, Substratlösung hinzugefügt (200 µl), die Reaktion durch Zugabe von 50 µl Stopp-Lösung unterbrochen und die Absorptionswerte, wie oben angegeben, bestimmt. Die Konzentrationen von TNF-BP I in den Proben wurden mit Hilfe des Titercalc-Programms (Hewlett Packard) berechnet.

In analoger Weise wurde ein Assay für die Analyse von Zellkulturüberständen aufgebaut, mit dem Unterschied, daß bei der Erstellung der Eichkurve Zellkulturmedium mit einem Gehalt von 10 % FCS eingesetzt und die Proben unverdünnt verwendet wurden.

Für die Bestimmung von TNF-BP I im Harn wurde eine modifizierte (zweistufige) Methode entwickelt. Die Notwendigkeit dafür ergab sich, weil der niedrige pH-Wert einiger Proben sowie andere, ungeklärte, Faktoren den Test störten. Der durch den pH-Wert bedingte Effekt konnte durch den Standard-Testpuffer aufgrund zu geringer Pufferkapazität nicht kompensiert werden. Es war ein starker Phosphatpuffer (0.5 M) erforderlich, um sicherzustellen, daß auch die sauersten Proben (pH 5) auf neutralen pH-Wert gebracht werden konnten. Diese Maßnahme war noch nicht ausreichend, weil einige Proben noch immer eine geringe Wiederfindung ergaben. Dieses Problem wurde durch Anwendung einer zweistufigen Testmethode (aufeinanderfolgende Inkubation von Proben und Konjugat) gelöst: Die Platten wurden beschichtet, blockiert und gewaschen, wie für den Serum-Assay beschrieben. Anschließend wurde eine Lösung, bestehend aus Rinderserumalbumin (5 g/l) und Tween 20 (0.5 g/l) in 0.5 molarem Natriumphosphatpuffer pH 7.4 in die Vertiefungen der Platte pipettiert (50 µl/Vertiefung). Die Eichkurve wurde mit derselben Lösung hergestellt. Daraufhin wurden Harnproben (50 µl/Vertiefung; 2fach-Bestimmung) hinzugefügt und die Platten auf einem Schüttelgerät 2 h lang inkubiert. Die Platten wurden anschließend gewaschen und 100 µl einer Lösung von Enzymkonjugat in Testpuffer zugegeben, worauf weitere 2 h inkubiert wurde. Die abschließende Behandlung der Platten und die quantitative Bestimmung von TNF-BP I wurden vorgenommen, wie oben für den Serumtest angegeben.

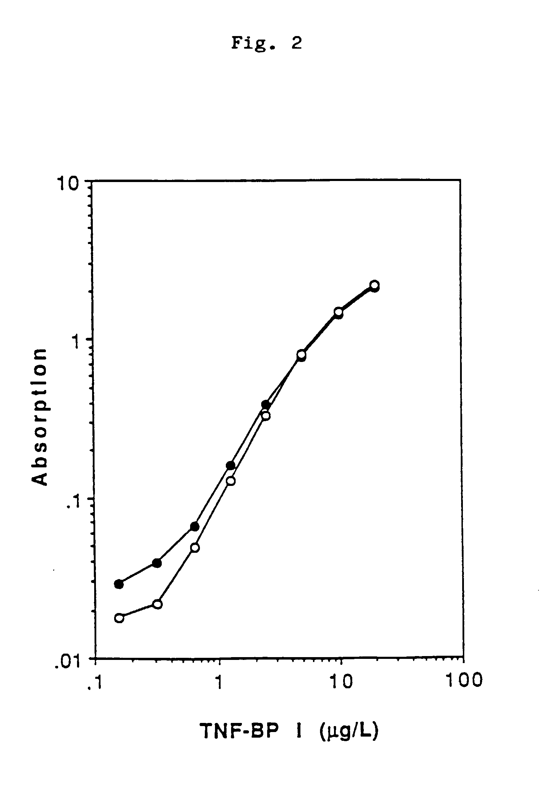

Eine typische Eichkurve ist in Fig. 2 dargestellt (gefüllte Kreise: Serumproben; offene Kreise: Harnproben); sie erlaubt eine quantitative Bestimmung von TNF-BP I in einem Konzentrationsbereich zwischen 0.3 und 10 µg/l. Die nachweisbare Mindestmenge im Serum, definiert als Konzentration, die ein Signal entsprechend dem Blindwertsignal plus drei Standardabweichungen ergibt, wurde mit 0.2 µg/l bestimmt (Mittelwert, 4 unabhängige Assays). Bei Konzentrationen bis zu 1 mg/l (100facher Überschuß gegenüber dem normalen Testbereich) konnte kein "high-dose hook"-Effekt (Antigen im Überschuß) festgestellt werden. Die Empfindlichkeit des Tests für Harnproben war vergleichbar. Die Empfindlichkeit für Zellkulturproben liegt im Bereich von 0.1 µg/l, da diese Proben unverdünnt eingesetzt werden.

Die Genauigkeit des Serum-Assays wurde überprüft, indem Serumproben mit einem Gehalt an TNF-BP I bei Konzentrationen von 2 und 10 µg/l in sechs verschiedenen Tests analysiert wurden. Die Intra-Assay-Variationskoeffizienten betrugen 4.7 % bzw. 5.9 %; die Inter-Assay-Variationskoeffizienten 6.6 % bzw. 7.5 %. Die Linearität wurde bestimmt, indem eine Serie von externen Zweifachverdünnungen von TNF-BP I im Serum (Konzentrationen zwischen 0.3 und 10 µg/l) untersucht und die lineare Regression der experimentell bestimmten Werte gegenüber den erwarteten Werten berechnet wurde. In drei unabhängigen Versuchen wurden Korrelationskoeffizienten zwischen 0.998 und 1 erhalten.

Die Wiederfindung von TNF-BP I mittels Serum-Assay wurde untersucht, indem exogenes TNF-BP I in zwei verschiedenen Konzentrationen (1 und 5 µg/l) dem Serum von 7 Normalspendern zugesetzt wurde. Dieser Versuch wurde dadurch erschwert, daß alle Proben endogenes TNF-BP I in verschiedenen Konzentrationen enthielten; diese Werte mußten daher vor der Berechnung der Wiederfindung abgezogen werden. Die durchschnittliche Wiederfindung wurde mit 83 ± 15 % bei 1 µg/l und mit 85 ± 13 % bei 5 µg/l (Bereich: 62 - 101 % bzw. 65 - 105 %) bestimmt.

Die Wiederfindung von TNF-BP I mittels des modifizierten Harn-Assays in 14 Harnproben, denen exogenes TNF-BP I zugesetzt wurde, wurde analog wie für die Serumproben untersucht; die durchschnittliche Wiederfindung bei einer nominalen Konzentration von 5 µg/l betrug 83 ± 15 % (Bereich: 52 - 104 %).

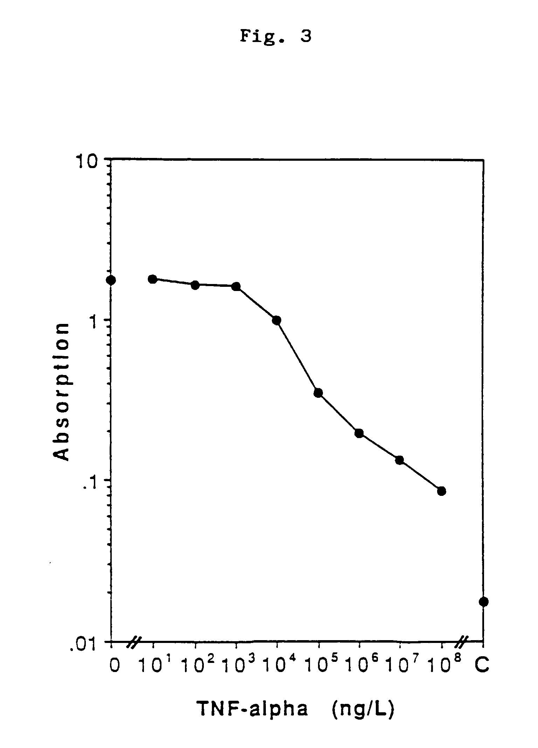

Um zu bestimmen, ob die physiologischen Liganden des TNF-Rezeptors die Wechselwirkung von TNF-BP I mit den Antikörpern beeinflussen, wurde der ELISA bei einer konstanten Konzentration von TNF-BP I (5 µg/l) in Gegenwart von zunehmenden Konzentrationen von rekombinantem TNF-α (Gray et al., 1984) und rekombinantem TNF-β (Pennica et al., 1984), jeweils aus *E. coli* und mit einer Reinheit von >99 %, durchgeführt. Wie sich aus Fig. 3 ergibt (in C ist der Assay-Untergrund bei Fehlen von TNF-BP I dargestellt), inhibiert TNF-α die Reaktivität von TNF-BP I mit den Antikörpern bei Konzentrationen ≥ 10 µg/l; im Gegensatz dazu zeigte TNF-β keine Wirkung auch bei sehr hohen Konzentrationen (bis zu 100 mg/l).

### Beispiel 4

### Stabilität von TNF-BP I

### a) Stabilität im Serum

Filtersterilisierte Proben von gepooltem Normalserum wurden 24 h lang bei verschiedenen Temperaturen gelagert; außerdem wurden die Proben mehreren Einfrier/Auftauzyklen unterworfen. Wie sich aus Tab. 1 ergibt, beeinträchtigten weder die Inkubation bei Temperaturen von 37°C noch das mehrfache Gefrieren und Wiederauftauen die Immunreaktivität von TNF-BP I. Die Proben bestanden aus gepooltem Humanserum mit einem Gehalt von endogenem TNF-BP I (Probe 1: 1.2 µg/l) und derselben Serumprobe mit Zusatz von exogenem TNF-BP I (Probe 2: Endkonzentration 5 µg/l).

### b) Stabilität im Harn

Die Versuche wurden durchgeführt, wie unter a) angegeben, wobei drei Proben untersucht wurden, die ein breites Spektrum von pH-Werten repräsentierten. Wie aus Tab. 1 hervorgeht, war TNF-BP I in allen Proben nach Gefrieren und Wiederauftauen stabil. Alle Proben konnten 24 h lang bei Temperaturen bis 37°C gelagert werden, ohne Aktivität einzubüßen. Die Harnproben stammten von drei verschiedenen Spendern (Probe 1: pH 5.1, 1.9 µg/l; Probe 2: pH 5.9, 4.0 µg/l; Probe 3: pH 7.2, 3.7 µg/l). "nd" bedeutet "nicht bestimmt".

### Beispiel 5

### Nachweis von TNF-BP I in Humanserum

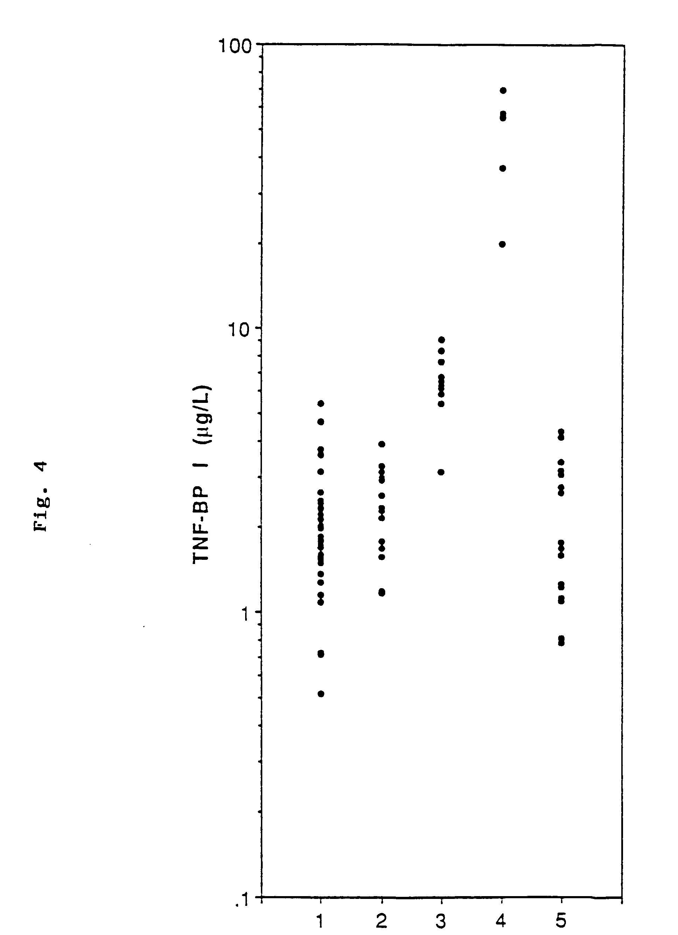

Seren von 42 normalen Spendern (Blutspender und Laborpersonal) wurden mit Hilfe des in Beispiel 3 beschriebenen Serum-Sandwich-ELISAs untersucht. TNF-BP I-Konzentrationen variierten zwischen 0.5 und 5.4 µg/l, bei einem Mittelwert von 2.1 µg/l (Standardabweichung 1.0 µg/l; Fig. 4). Von zwei Personen waren Serum, EDTA-Plasma, Citratplasma und Heparinplasma, gewonnen zum selben Zeitpunkt, verfügbar; die TNF-BP I-Konzentrationen unterschieden sich nicht signifikant (Spender A: 1.7, 2.0, 1.6 und 1.9 µg/l; Spender B: 1.8, 1.8, 1.8 und 1.9 µg/l). Die TNF-BP I-Konzentrationen in Seren von 15 Patienten mit chronischer Polyarthritis unterschieden sich nicht signifikant von denen gesunder Personen (2.3 ± 0.79 µg/l; Bereich: 1.2 - 3.9 µg/l). Signifikant erhöhte Werte wurden in Seren von Patienten mit schweren Verbrennungen gefunden (6.5 ± 1.7 µg/l; Bereich: 3.1 - 9.1 µg/l; n = 10). Patienten mit Nierenversagen (n = 6) zeigten bemerkenswert hohe Konzentrationen in einem Bereich von 20 - 69 µg/l (Mittelwert ± Standardabweichung: 49 ± 17 µg/l).

### Beispiel 6

Unter Verwendung des in Beispiel 3 spezifisch für Harnproben entwickelten Sandwich-ELISA wurden die TNF-BP I-Konzentrationen in Harnproben von 16 Normalspendern bestimmt. Sie variierten zwischen 0.78 und 4.3 µg/l (Mittelwert ± Standardabweichung: 2.2 ± 1.2 µg/l). Es gab keinen signifikanten Unterschied zwischen weiblichen (2.1 ± 1.4 µg/l; n = 9) und männlichen (2.2 ± 1.0 µg/l; n = 7) Spendern.

### Beispiel 7

### Bestimmung von TNF-BP I in Kulturüberständen von humanen Zellinien

Um festzustellen, ob der entwickelte ELISA sich für den Nachweis von TNF-BP I eignet, das von humanen Zellkulturen produziert wird, wurde eine Reihe von Zellinien untersucht. Kulturmedien (mit einem Gehalt von 10 % FCS) wurden von dichten Kulturen geerntet, im allgemeinen mehrere Tage nach Verdünnung mit frischem Medium oder nach Passage von adhärenten Zellen.

Kulturmedium mit einem Gehalt von 10 % FCS wurde als Standardverdünnungsmittel verwendet; die Assays wurden in der einstufigen Version durchgeführt. TNF-BP I war in Überständen der Zellinien A549 (Lungenkarzinom; 1.1 µg/l), HeLa (Zervixkarzinom; 0.38 µg/l) und Namalwa (Burkitt-Lymphom; 0.25 µg/l) nachweisbar, lag jedoch unterhalb der Nachweisgrenze (100 ng/l) in den Zellinien U937 (Histiozyten-Lymphom), EoL-3 (eosinophile Leukämie), Raji (Burkitt-Lymphom), HL-60 (myeloide Leukämie), U266 (Myelom) und LuKII (B-lymphoblastoide Zellinie, immortalisiert durch Epstein-Barr Virus). In Übereinstimmung mit früheren Ergebnissen (Lantz et al., 1990b) wurde beobachtet, daß HL-60-Zellen, die in Gegenwart von TNF-β (10 µg/l) kultiviert wurden, erhöhte Mengen von TNF-BP I freisetzten; nach 4 Tagen dieser Behandlung wurde eine Konzentration von 0.45 µg/l erreicht.

### Beispiel 8

### a) Bestimmung des Einflusses von TNF-α auf die Bindung der Antikörper an TNF-BP I

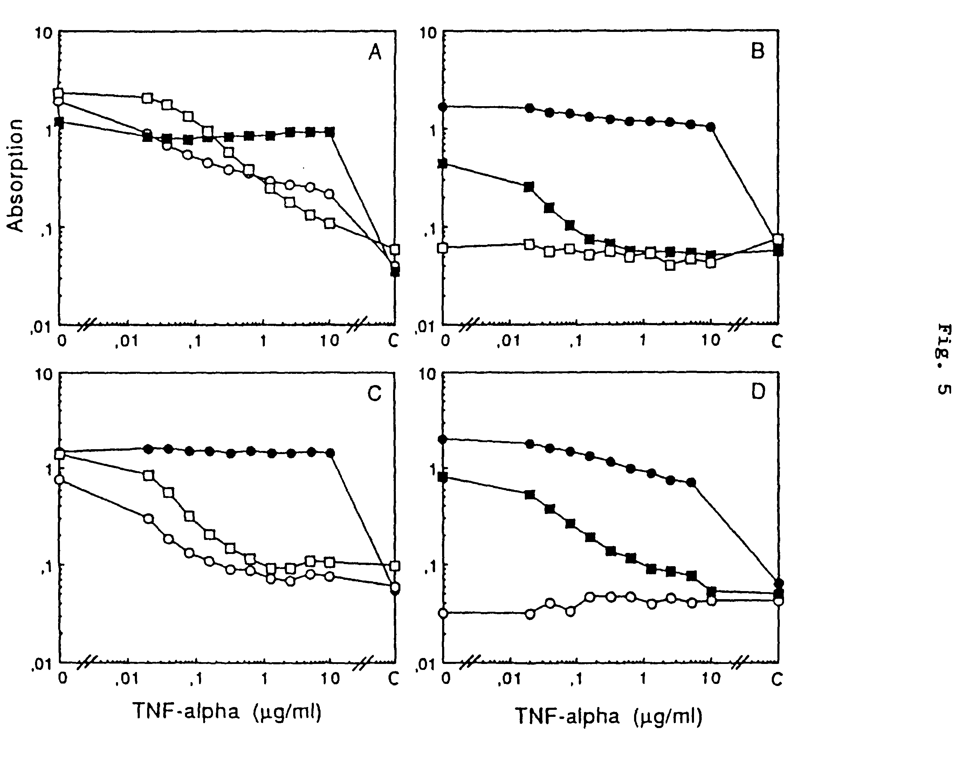

Um festzustellen, ob TNF-α die Bindung der Antikörper an TNF-BP I beeinträchtigt, wurde ein einstufiger Sandwich-ELISA in mehreren verschiedenen Anordnungen durchgeführt. Dabei wurde jeweils einer der monoklonalen Antikörper als Beschichtungsantikörper zum Einfangen des Antigens, eine konstante Menge von TNF-BP I in Gegenwart von unterschiedlichen TNF-α-Konzentrationen und ein zweiter monoklonaler Antikörper, markiert mit Meerrettich-Peroxidase, verwendet. Die ELISAs wurden im wesentlichen durchgeführt, wie in Beispiel 3 beschrieben: Die Platten wurden mit 10 µg/ml Antikörper in Beschichtungspuffer beschichtet, gewaschen und mit Testpuffer blockiert. Die einstufige Inkubation mit TNF-BP I in einer Endkonzentration von 5 ng/ml (in Gegenwart unterschiedlicher Konzentrationen von TNF-α) und mit Peroxidase-gekoppeltem Antikörper wurde in Testpuffer bei Raumtemperatur während 3 h durchgeführt. Die Platten wurden daraufhin gewaschen, mit Substratlösung entwickelt, die Reaktion gestoppt und die Absorption bei 450 nm in einem Plattenleser gemessen, wobei die Absorption bei 690 nm abgezogen wurde. Das Ergebnis dieser Versuche ist in Fig. 5 dargestellt: Die Tafeln A - D zeigen die Ergebnisse von Platten, die mit den Antikörpern tbp-1, tbp-2, tbp-6 bzw. H398 beschichtet waren; die Symbole stellen die Peroxidase-Konjugate der Antikörper tbp-1 (geschlossene Kreise), tbp-2 (offene Kreise), tbp-6 (geschlossene Vierecke) bzw. H398 (offene Vierecke) dar; bei C ist das Background-Signal (Abwesenheit von TNF-BP I) aufgetragen. Es zeigte sich, daß das mit Kombinationen der Antikörper tbp-1 und tbp-6 erhaltene Signal nicht einmal durch die höchsten TNF-α-Konzentrationen, die getestet wurden (10 µg/ml), beeinflußt wurde. Im Gegensatz dazu waren alle Kombinationen, die tbp-2 oder H398 enthielten, empfindlich auf TNF-α, wenn auch mit verschiedenen Dosis-Wirkung-Verhältnissen. (Diese Ergebnisse deuten darauf hin, daß H398 und tbp-2 Epitope erkennen, die mit der TNF-α-Bindungsstelle in Zusammenhang stehen, während tbp-1 und tbp-6 davon unabhängige Epitope definieren.)

### b) Bestimmung des Einflusses von TNF-β auf die Bindung der Antikörper an TNF-BP I

Der Assay wurde mit den monoklonalen Antikörpern tbp-1 und tbp-6 für TNF-β durchgeführt, wie in a) beschrieben. Auch für TNF-β zeigte sich, daß Konzentrationen von 10 µg/ml den Assay nicht stören.

### Beispiel 9

### Zytotoxischer Bioassay zur Bestimmung der biologischen Aktivität der monoklonalen Antikörper

### a) Einfluß der Antikörper auf die Schutzwirkung von TNF-BP I gegenüber TNF-α

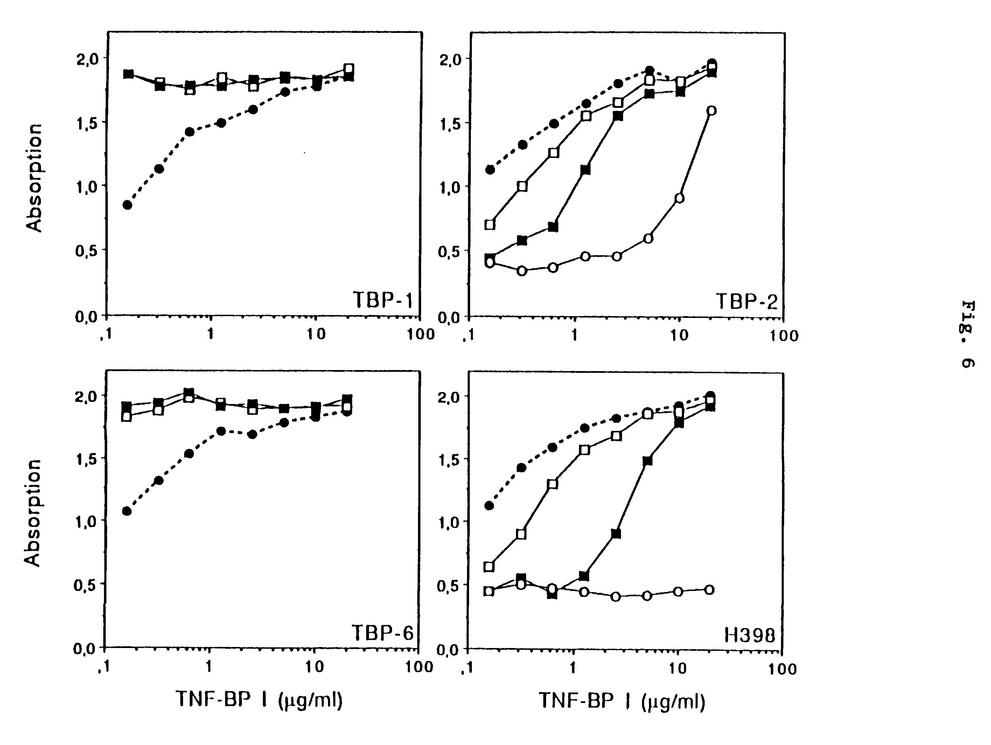

Die zytotoxische Aktivität von TNF-α wurde im wesentlichen nach der von Kramer und Carver, 1986, beschriebenen Methode bestimmt. Dazu wurden Maus-L-M-Zellen (ATCC CCL 1.2) über Nacht in Mikrotiterplatten gezüchtet, TNF-α-Präparationen wurden in seriellen Zweifachverdünnungen aufgegeben und Actinomycin D bei einer Endkonzentration von 1 µg/ml zugegeben. Die Platten wurden 18 bis 20 h lang bei 39°C inkubiert, die Zellen wurden mit 0.5 % Kristallviolett gefärbt und die Absorption bei 540 nm bestimmt. (Zell-Kontrollen und Leerwerte wurden auf jeder Platte 4-fach vorgesehen.) Definitionsgemäß enthält eine Lösung, die die Abtötung von 50 % der Zellen bewirkt, 1 Einheit/ml. Unter den Assaybedingungen betrug die spezifische Aktivität des eingesetzten rekombinanten humanen TNF-α 5 x 107 Einheiten/mg Protein; das Referenzpräparat für TNF-α, 86/659 (NIBSC, England) mit einer festgesetzten Aktivität von 4 x 104 E/ml, zeigte eine Aktivität von 5 x 104 E/ml. Die Neutralisierungsassays wurden durchgeführt, indem Serienverdünnungen von TNF-BP I in Kulturmedium mit einer konstanten Menge TNF-α (Endkonzentration 20 E/ml) und/oder monoklonalen Antikörpern 1 h bei 37°C vorinkubiert wurden. Daraufhin wurde die Kulturflüssigkeit von der Platte entfernt und die vorinkubierte TNF-α/TNF-BP I/Antikörper-Lösung den Zellen in einer Menge von 100 µl beigegeben. Die Platten wurden inkubiert und gefärbt, wie oben beschrieben. TNF-BP I-Konzentrationen, die eine Schutzwirkung von 50 % auf die Zellen zeigten (ED50), wurden graphisch ermittelt. Das Ergebnis des durchgeführten Bioassays ist in Fig. 6 dargestellt; die Symbole stellen folgende Ergebnisse dar: TNF-BP I allein (gefüllte Kreise; durchbrochene Linie), TNF-BP I in Kombination mit den Antikörpern bei 0.01 µg/ml (offene Dreiecke), 0.1 µg/ml (gefüllte Dreiecke), 1 µg/ml (offene Vierecke), 10 µg/ml (gefüllte Vierecke) oder 100 µg/ml (offene Kreise), dar.

Es wurde festgestellt, daß in Gegenwart einer konstanten Menge von TNF-α (400 pg/ml entsprechend 20 zytotoxischen Einheiten/ml) halbmaximaler Schutz bei TNF-BP I-Konzentrationen von 270 ± 44 ng/ml (Mittelwert von 6 unabhängigen Tests) eintritt. Wenn der monoklonale Antikörper tbp-2 zusammen mit TNF-BP I zugegeben wurde, wurde die Schutzwirkung von TNF-BP I vermindert: Bei Antikörperkonzentrationen von 1, 10 oder 100 µg/ml erhöhten sich die für den halbmaximalen Schutz erforderlichen TNF-BP I-Konzentrationen auf 380, 1.000 bzw. 12.000 ng/ml. H398 verminderte die Schutzwirkung von TNF-BP I in ähnlichem Maße (Endwerte von 840, 3.800 und > 20.000 ng/ml). (Die Behandlung der Zellen mit diesen Antikörpern in Mengen bis zu 100 µg/ml in Abwesenheit von TNF ergab keinerlei zytotoxische Wirkungen; darüberhinaus wurde festgestellt, daß die Antikörper die Zellen in Abwesenheit von TNF-BP I nicht gegen die Toxizität von TNF schützen.)

Der Antikörper tbp-1 erhöhte hingegen überraschenderweise die Schutzwirkung von TNF-BP I. Bei Antikörperkonzentrationen hinunter bis zu 100 ng/ml wurden die Zellen durch TNF-BP I bei so niedrigen Dosen wie 40 ng/ml vollständig geschützt. tbp-6 zeigte ähnliche, aber quantitativ geringere Aktivität (dieser Antikörper zeigte auch in den ELISAs geringere Aktivität und hat vermutlich geringere Affinität). In Gegenwart von großem Antikörperüberschuß (10 µg/ml) konnte halbmaximaler Schutz bei TNF-BP I-Konzentrationen von 7.3 ± 0.5 ng/ml (tbp-1) bzw. 53 ± 13 ng/ml (tbp-6), entsprechend einer ca. 40-fachen bzw. 5-fachen Verstärkung der Schutzwirkung (dazu wurden Titrationen in 4 Versuchen durchgeführt), festgestellt werden. In Kontrollversuchen, die bei derselben TNF-α-Konzentration in Abwesenheit von TNF-BP I durchgeführt wurden, zeigten die Antikörper keine Wirkung.

### b) Einfluß der Antikörper auf die Schutzwirkung von TNF-BP I gegenüber TNF-β

In dem Bioassay mit L-M-Zellen, der wie in a) beschrieben durchgeführt wurde, ist die zytotoxische Aktivität von TNF-β höher als die von TNF-α (300 E/ng gegenüber 50 E/ng). TNF-BP I zeigte gegen dieselbe zytotoxische Dosis von TNF-β (20 E/ml entsprechend 66 pg/ml) ebenfalls eine Hemmung des zytotoxischen Effektes, es wurden aber mindestens zehnmal so hohe Dosen benötigt (ED50 = 3.800 ng/ml, verglichen mit 270 ng/ml für TNF-α). Die Zugabe der Antikörper tbp-1 und tbp-6 (10 µg/ml) verstärkte die Schutzwirkung in einem ähnlichen Ausmaß wie für TNF-α; halbmaximale Schutzwirkung wurde bei 53 ng/ml bzw. 500 ng/ml erzielt.

**TABELLE 1**

| Stabilität von TNF-BP I in Serum und Harn | | | | | |
|---|---|---|---|---|---|
| Behandlung | | | % Wiederfindung | | |
| | Serum | | Harn | | |
| | Probe 1 | Probe 2 | Probe 1 | Probe 2 | Probe 3 |
| Lagerung bei | | | | | |
| -20°C | 100 | 100 | 100 | 100 | 100 |
| + 4°C | 98 | 100 | 97 | 105 | 105 |
| +20°C | 100 | 98 | 100 | 112 | 109 |
| +37°C | 98 | 91 | 110 | 107 | 89 |

| Gefrier/Auftauzyklen | | | | | |
|---|---|---|---|---|---|
| 0 | 100 | 100 | 100 | 100 | 100 |
| 1 | 101 | 105 | 99 | 92 | 101 |
| 3 | 95 | 103 | 107 | 98 | 99 |
| 5 | 92 | 104 | nd | 94 | 99 |

### Literatur:

Beutler B., 1988, Am. J. Med. 85, 287-288
Beutler B. und Cerami A., 1989, Ann. Rev. Immunol. 7, 625-655
Brockhaus M. et al., 1990, Proc. Natl. Acad. Sci. USA 87, 3127-3131
Engelmann H. et al., 1989, J. Biol. Chem. 264, 11974-11980
Engelmann H. et al., 1990 a, J. Biol. Chem. 265, 1531-1536
Engelmann H. et al., 1990 b, J. Biol. Chem. 265, 14497-14504
Gray P.W. et al., 1984, Nature 312, 721-724
Himmler A. et al., 1990, DNA Cell Biol. 9, 705-715
Kearney J.F. et al., 1979, J. Immunology 123, 1548-1550
Köhler G. und Milstein C., 1975, Nature 265, 495-497
Kohno T. et al., 1990, Proc. Natl. Acad. Sci. USA 87, 8331-8335
Kramer, S.M. und Carver, M.E., 1986, J. Immunol. Meth. 93, 201-206
Lähdevirta J. et al., 1988, Am. J. Med. 85, 289-291
Lantz M. et al., 1990 a, Cytokine 2, 1-5
Lantz M. et al., 1990 b, J. Clin. Invest. 86, 1396-1402
Loetscher H. et al., 1990, Cell 61, 351-359
Loetscher H. et al., 1991, J. Biol. Chem. 266, 18324-18329
Michie H.R. et al., 1988, J. Medicine 318, 1481-1486
Offner F. et al., 1990, J. Lab. Clin. Med. 116, 100-105
Olsson I. et al., 1989, Eur. J. Haematol. 42, 270-275
Paul N.L. und Ruddle N., 1988, Ann. Rev. Immunol. 6, 407-438
Peetre C. et al., 1988, Eur. J. Haematol. 41, 414-419
Pennica D. et al., 1984, Nature 312, 724-728
Schall T.J. et al., 1990, Cell 61, 361-370
Seckinger P. et al., 1988, J. Exp. Med. 167, 1511-1516
Smith C.A. et al., 1990, Science 248, 1019-1023
Thoma B. et al., 1990, J. Exp. Med. 172, 1019-1023
Wilson M.B. und Nakane P.K., 1978, Immunfluorescence and Related Staining Techniques, Elsevier-North Holland, Amsterdam, 215-224

## Patentansprüche

1. Monoklonale Antikörper gegen humanes TNF-BP I mit der Bezeichnung tbp-1, sezerniert von der Hybrodomzelline TBP-1 (ECACC 91060555), und tbp-6, sezerniert von der Hybrodomzelline TBP-6(ECACC 91112811), oder aktive Fragmente davon.

2. Hybridomzellinien TBP-1 und TBP-6, hinterlegt bei der ECACC unter den Hinterlegungsnummern 91060555 (TBP-1) bzw. 91112811 (TBP-6).

3. Verfahren zur Bestimmung von TNF-BP I in einer Probe, dadurch gekennzeichnet, daß die Probe mit dem Antikörper tbp-1 gemäß Anspruch 1 in Kontakt gebracht wird und die Bildung eines binären oder ternären Komplexes zwischen dem in der Probe enthaltenen TNF-BP I und tbp-1 bestimmt wird.

4. Verfahren zur Bestimmung von TNF-BP I in einer Probe, dadurch gekennzeichnet, daß die Bildung eines ternären Antikörper/Antigen/Antikörper-Komplexes bestimmt wird, wobei als erster Antikörper tbp-1 gemäß Anspruch 1 und als zweiter Antikörper ein Antikörper, der in der Lage ist,mit tbp-1 einen Antikörper/Antigen/Antikörper-Komplex zu bilden, eingesetzt wird.

5. Verfahren nach Anspruch 4, dadurch gekennzeichnet, daß als zweiter Antikörper tbp-2, sezerniert von der Hybrodomzelline TBP-2 (ECACC 91060556), eingesetzt wird.

6. Verfahren nach einem der Ansprüche 3 bis 5, dadurch gekennzeichnet, daß die Probe eine Körperflüssigkeit ist.

7. Verfahren nach Anspruch 6, dadurch gekennzeichnet, daß die Körperflüssigkeit Serum ist.

8. Verfahren nach Anspruch 6, dadurch gekennzeichnet, daß die Körperflüssigkeit Plasma ist.

9. Verfahren nach Anspruch 6, dadurch gekennzeichnet, daß die Körperflüssigkeit Harn ist.

10. Verfahren nach einem der Ansprüche 3 bis 5, dadurch gekennzeichnet, daß die Probe ein Zellkulturüberstand ist.

11. Verfahren nach einem der Ansprüche 4 bis 10, dadurch gekennzeichnet, daß
a) die Probe mit trägergebundenem tbp-1 gemäß Anspruch 1 in Kontakt gebracht wird,
b) der in a) gebildete Komplex mit dem in Anspruch 5 definierten Antikörper tbp-2 oder einem Antikörper, der in der Lage ist, mit tbp-1 einen Antikörper/Antigen/Antikörper-Komplex zu bilden, in Berührung gebracht wird, wobei der in diesem Schritt eingesetzte Antikörper eine meßbare Markierung aufweist,
c) die Menge des gebildeten Antikörper/Antigen/Antikörper-Komplexes durch Messung der Markierung bestimmt wird.

12. Verfahren nach Anspruch 11, dadurch gekennzeichnet, daß in b) ein Antikörper eingesetzt wird, der dasselbe Epitop von TNF-BP I oder einen Bereich davon erkennt wie tbp-2.

13. Verfahren nach Anspruch 11 oder 12, dadurch gekennzeichnet, daß in b) ein Enzym-gekoppelter Antikörper eingesetzt wird.

14. Anwendung des Verfahrens nach einem der Ansprüche 6-8 zur Diagnose von pathologischen Zuständen des menschlichen Körpers, die mit einer Aktivierung des TNF-Rezeptorsystems verbunden sind, insbesondere von Zuständen, bei denen die TNF-α-Konzentration rascher absinkt als die TNF-BP I-Konzentration.

15. Anwendung nach Anspruch 14 zur Diagnose von septischem Schock.

16. Sandwich-Immunoassay-Kit zur Durchführung des Verfahrens nach einem der Ansprüche 4 bis 13, dadurch gekennzeichnet, daß er in einem Behälter den Antikörper tbp-1 gemäß Anspruch 1 und in einem weiteren Behälter den in Anspruch 5 definierten Antikörper tbp-2 enthält, wobei einer der beiden Antikörper gegebenenfalls an einen festen Träger gebunden ist und wobei der jeweils andere Antikörper eine meßbare Markierung aufweist und wobei gegebenenfalls tbp-2 durch einen anderen Antikörper ersetzt ist, der die Fähigkeit aufweist, mit tbp-1 einen Antikörper/Antigen/Antikörper-Komplex zu bilden.

17. Kit nach Anspruch 16, dadurch gekennzeichnet, daß der tbp-2 ersetzende Antikörper an dasselbe oder an ein überlappendes Epitop von TNF-BP I bindet wie tbp-2.

18. Verfahren zur Bestimmung von TNF-BP I in einer Probe, dadurch gekennzeichnet, daß die Probe mit den Antikörpern tbp-1 und tbp-6 gemäß Anspruch 1 in Kontakt gebracht und die Bildung eines ternären Komplexes zwischen dem in der Probe enthaltenen TNF-BP I und den Antikörpern bestimmt wird.

19. Verfahren nach Anspruch 18, dadurch gekennzeichnet, daß
a) die Probe mit trägergebundenem tbp-1 oder tbp-6 in Kontakt gebracht wird,
b) der in a) gebildete Komplex mit tbp-6 oder mit tbp-1 in Berührung gebracht wird, wobei der in diesem Schritt eingesetzte Antikörper eine meßbare Markierung aufweist,
c) die Menge des gebildeten Antikörper/Antigen/Antikörper-Komplexes durch Messung der Markierung bestimmt wird.

20. Sandwich-Immunoassay-Kit zur Durchführung des Verfahrens nach Anspruch 19, dadurch gekennzeichnet, daß er in einem Behälter den Antikörper tbp-1 gemäß Anspruch 1 und in einem weiteren Behälter den Antikörper tbp-6 gemäß Anspruch 1 enthält, wobei einer der beiden Antikörper gegebenenfalls an einen festen Träger gebunden ist und wobei der jeweils andere Antikörper eine meßbare Markierung aufweist.

21. Verwendung der monoklonalen Antikörper tbp-1 und/oder tbp-6 gemäß Anspruch 1 zur Verstärkung der Schutzwirkung von endogenem oder exogenem TNF-BP I gegen TNF-α und/oder TNF-β.

## Claims

1. Monoclonal antibodies against human TNF-BP I entitled tbp-1, secreted by the hybridoma cell line TBP-1 (ECACC 91060555) and tbp-6, secreted by the hybridoma cell line TBP-6 (ECACC 91112811), or active fragments thereof.

2. Hybridoma cell lines TBP-1 and TBP-6 deposited at the ECACC under deposit numbers 91060555 (TBP-1) and 91112811 (TBP-6), respectively.

3. Process for determining TNF-BP I in a sample, characterised in that the sample is brought into contact with the antibody tbp-1 according to claim 1 and the formation of a binary or ternary complex between the TNF-BP I contained in the sample and the tbp-1 is determined.

4. Process for determining TNF-BP I in a sample, characterised in that the formation of a ternary antibody/antigen/antibody complex is determined, the first antibody used being tbp-1 according to claim 1 and the second antibody used being an antibody with the ability to form an antibody/antigen/antibody complex with tbp-1.

5. Process according to claim 4, characterised in that tbp-2, secreted by the hybridoma cell line TBP-2 (ECACC 91060556) is used as the second antibody.

6. Process according to one of claims 3 to 5, characterised in that the sample is a body fluid.

7. Process according to claim 6, characterised in that the body fluid is serum.

8. Process according to claim 6, characterised in that the body fluid is plasma.

9. Process according to claim 6, characterised in that the body fluid is urine.

10. Process according to one of claims 3 to 5, characterised in that the sample is a cell culture residue.

11. Process according to one of claims 4 to 10, characterised in that
a) the sample is brought into contact with carrier-bound tbp-1 according to claim 1,
b) the complex formed in a) is brought into contact with the antibody tbp-2 defined in claim 5, which is capable of forming an antibody/antigen/antibody complex with tbp-1, whilst the antibody used in this step has a measurable label,
c) the quantity of antibody/antigen/antibody complex formed is determined by measuring the label.

12. Process according to claim 11, characterised in that, in b) an antibody is used which recognises the same epitope of TNF-BP I, or a region thereof, as tbp-2.

13. Process according to claim 11 or 12, characterised in that in b) an enzyme-coupled antibody is used.

14. Use of the process according to one of claims 6 to 8 for diagnosing pathological conditions of the human body which involve activation of the TNF-receptor system, particularly conditions in which the TNF-α concentration falls more rapidly than the TNF-BP I concentration.

15. Use according to claim 14 for diagnosing septic shock.

16. Sandwich immunoassay kit for carrying out the process according to one of claims 4 to 13,
characterised in that it contains tbp-1 according to claim 1 in one container and the antibody tbp-2 as defined in claim 5 in another container, one of the two antibodies optionally being bound to a solid carrier, whilst the other antibody has a measurable label and, optionally, tbp-2 is replaced by another antibody which has the ability to form an antibody/antigen/antibody complex with tbp-1.

17. Kit according to claim 16, characterised in that the antibody replacing tbp-2 binds to the same epitope or to an overlapping epitope of TNF-BP I as tbp-2.

18. Process for determining TNF-BP I in a sample, characterised in that the sample is brought into contact with the antibodies tbp-1 and tbp-6 according to claim 1 and the formation of a ternary complex between the TNF-BP I contained in the sample and the antibodies is determined.

19. Process according to claim 18, characterised in that
a) the sample is brought into contact with carrier-bound tbp-1 or tbp-6,
b) the complex formed in a) is brought into contact with tbp-6 or with tbp-1, the antibody used in this step having a measurable label,
c) the quantity of antibody/antigen/antibody complex formed is determined by measuring the label.

20. Sandwich immunoassay kit for carrying out the process according to claim 19, characterised in that it contains the antibody tbp-1 according to claim 1 in one container and the antibody tbp-6 according to claim 1 in another container, whilst one of the two antibodies is optionally bound to a solid carrier and the other antibody has a measurable label.

21. Use of the monoclonal antibodies tbp-1 and/or tbp-6 according to claim 1 for intensifying the protective effect of endogenous or exogenous TNF-BP I against TNF-α and/or TNF-β.

## Revendications

1. Anticorps monoclonaux contre la TNF-BP I humaine ayant la désignation tbp-1, sécrété par la lignée cellulaire d'hybridome TBP-1 (ECACC 91060555), et tbp-6, sécrété par la lignée cellulaire d'hybridome TBP-6 (ECACC 91112811), ou fragments actifs de ceux-ci.

2. Lignées cellulaires d'hybridome TBP-1 et TBP-6, déposées auprès de la ECACC sous les numéros de dépôt 91060555 (TBP-1) et 91112811 (TBP-6).

3. Procédé de détermination de TNF-BP I dans un échantillon caractérisé en ce que l'échantillon est mis en contact avec l'anticorps tbp-1 selon la revendication 1 et la formation d'un complexe binaire ou ternaire entre la TNF-BP I contenue dans l'échantillon et tbp-1 est déterminée.

4. Procédé de détermination de TNF-BP I dans un échantillon caractérisé en ce que la formation d'un complexe ternaire anticorps/antigène/anticorps est déterminée, où tbp-1 selon la revendication 1 est utilisé comme premier anticorps et un anticorps qui est en mesure de former un complexe anticorps/antigène/anticorps avec tbp-1 est utilisé comme second anticorps.

5. Procédé selon la revendication 4 caractérisé en ce que tbp-2, sécrété par la lignée cellulaire d'hybridome TBP-2 (ECACC 91060556), est utilisé comme second anticorps.

6. Procédé selon l'une des revendications 3 à 5 caractérisé en ce que l'échantillon est un liquide biologique.

7. Procédé selon la revendication 6 caractérisé en ce que le liquide biologique est le sérum.

8. Procédé selon la revendication 6 caractérisé en ce que le liquide biologique est le plasma.

9. Procédé selon la revendication 6 caractérisé en ce que le liquide biologique est l'urine.

10. Procédé selon l'une des revendications 3 à 5 caractérisé en ce que l'échantillon est un surnageant de culture cellulaire.

11. Procédé selon l'une des revendications 4 à 10 caractérisé en ce que:
a) l'échantillon est mis en contact avec tbp-1 selon la revendication 1 lié à un support,
b) le complexe formé en a) est mis en contact avec l'anticorps tbp-2 défini dans la revendication 5 ou un anticorps qui est en mesure de former un complexe anticorps/antigène/anticorps avec tbp-1, où l'anticorps utilisé dans cette étape comporte un marqueur mesurable,
c) la quantité de complexe anticorps/antigène/anticorps formé est déterminée par mesure du marqueur.

12. Procédé selon la revendication 11 caractérisé en ce qu'un anticorps qui reconnaît le même épitope de TNF-BP I que tbp-2, ou un domaine de celui-ci, est utilisé en b).

13. Procédé selon la revendication 11 ou 12 caractérisé en ce qu'un anticorps couplé à une enzyme est utilisé en b).

14. Application du procédé selon l'une des revendications 6-8 pour le diagnostic d'états pathologiques du corps humain qui sont liés à une activation du système des récepteurs de TNF, en particulier d'états dans lesquels la concentration de TNF-α diminue plus vite que la concentration de TNF-BP I.

15. Application selon la revendication 14 pour le diagnostic du choc septique.

16. Kit d'immunodosage de type sandwich pour la mise en oeuvre du procédé selon l'une des revendications 4 à 13 caractérisé en ce qu'il contient dans un récipient l'anticorps tbp-1 selon la revendication 1 et dans un autre récipient l'anticorps tbp-2 défini dans la revendication 5, où l'un des deux anticorps est éventuellement lié à un support solide et où l'autre anticorps comporte un marqueur mesurable et où tbp-2 est éventuellement remplacé par un autre anticorps qui présente l'aptitude à former un complexe anticorps/antigène/anticorps avec tbp-1.

17. Kit selon la revendication 16 caractérisé en ce que l'anticorps remplaçant tbp-2 se lie au même épitope de TNF-BP I que tbp-2 ou à un épitope chevauchant.

18. Procédé de détermination de TNF-BP I dans un échantillon caractérisé en ce que l'échantillon est mis en contact avec les anticorps tbp-1 et tbp-6 selon la revendication 1 et la formation d'un complexe ternaire entre la TNF-BP I contenue dans l'échantillon et les anticorps est déterminée.

19. Procédé selon la revendication 18 caractérisé en ce que:
a) l'échantillon est mis en contact avec tbp-1 ou tbp-6 lié à un support,
b) le complexe formé en a) est mis en contact avec tbp-6 ou avec tbp-1, où l'anticorps utilisé dans cette étape comporte un marqueur mesurable,
c) la quantité de complexe anticorps/antigène/anticorps formé est déterminée par mesure du marqueur.

20. Kit d'immunodosage de type sandwich pour la mise en oeuvre du procédé selon la revendication 19 caractérisé en ce qu'il contient dans un récipient l'anticorps tbp-1 selon la revendication 1 et dans un autre récipient l'anticorps tbp-6 selon la revendication 1, où l'un des deux anticorps est éventuellement lié à un support solide et où l'autre anticorps comporte un marqueur mesurable.

21. Utilisation des anticorps monoclonaux tbp-1 et/ou tbp-6 selon la revendication 1 pour renforcer l'effet protecteur de TNF-BP I endogène ou exogène contre TNF-α et/ou TNF-β.
